# EUROPEAN PATENT APPLICATION

(11) **EP 3 056 562 A1**
(43) Date of publication of application: **17.08.2016**
(21) Application number: 16155308.6
(22) Date of filing: 11.02.2016
(51) Int. Cl.: C12N 5/071

(54) **INDUCED SMOOTH MUSCLE-TYPE CELLS, METHODS FOR THE PRODUCTION THEREOF, AND USES THEREOF IN CELL-BASED TISSUE REGENERATION AND TREATMENT OF VASCULAR OCCLUSION**

(30) Priority: 11.02.2015 EP 15154636
(71) Applicant: University College Cork-National University of Ireland, Cork, Cork (IE)
(72) Inventor: Caplice, Noel, Co. Cork (IE)
(74) Representative: Purdy, Hugh Barry

(57) **Abstract**

An induced smooth muscle-like cell (iSMC) is described. The iSMC is prepared by reprogramming a primitive progenitor cell comprising introducing into the cell an agent capable of increasing the expression of myocardin in the primitive progenitor cell. The agent may be a nucleic acid construct comprising a KLF4 knockdown agent, an artificial myocardinspecific transactivator, or a myocardin transgene typically under the control of a transcriptional promotor. Methods of producing the cells, and the use of the cells in autologous tissue regeneration therapy or treatment of vascular occlusion in humans is also described.

## Description

### Introduction

Smooth muscle cell (SMC) loss or dysfunction affects multiple tissues in the body contributing to a spectrum of cardiovascular disorders including atherosclerosis, systemic and pulmonary hypertension, myocardial infarction, and heart failure and to hollow organ disease of the respiratory, gastro-intestinal and genito-urinary tracts. Together these diverse pathologies afflict tens of millions of people worldwide on an annual basis. Regenerative and replacement therapies in these conditions have been restricted largely due to use of allogeneic adult SMC or limited expansion capacity of SMC derived from stem/progenitor cells described to date. Thus cells and technologies that generate scalable and autologous sources of SMC are of great interest as they would enable expanded smooth muscle tissue and organ replacement as well as molecular, genetic and drug screening of conditions where SMC are the primary cellular focus of disease aberrancy. Methodology providing highly expandable progenitor cells from an easily accessible source with lineage reprogramming potential may meet these requirements.

In embryos, fate-mapping studies have identified multiple sources of smooth muscle progenitor cells, each having a different lineage but transcribing a common set of smooth muscle markers upon differentiation. Unfortunately, although embryonic and induced pluripotent stem cells (iPSC) can be used to generate SMC, sufficient scale up of these differentiated cells to enable tissue replacement has not be demonstrated to date. Recently, reprogramming of somatic cells has highlighted the importance of understanding the molecular pathways that specify lineage and differentiation of stem/progenitor cells. In adults, iPSC technology can be used to generate SMC but induced pluripotency in general may also be restricted in terms of emerging immunogenic and off-target responses to lineage specific cell derivatives. Adult tissue resident, bone marrow and circulating sources of smooth muscle progenitors exist but each has technical limitations whether it be replicative senescence on expansion, restricted access as in cardiac-and vessel wall-derived progenitors or restricted differentiation capacity in the case of progenitors from hematogenous sources. Moreover the lack of established methodologies to identify smooth muscle progenitors, other than ability to differentiate into SMC-like cells in culture or *in vivo,* has meant that the isolation of a clonogenic adult smooth muscle progenitor cell with the potential to efficiently differentiate into scalable functional SMCs remains elusive.

Kumar et al (Biomaterials 35 (2014) 9012-9022) describes smooth muscle cells engineered to deliver high local concentrations of VEGF *in-vivo*, and describes the delivery of the engineered cells as part of an intravascular cell delivery device for therapeutic VEGF-induced angiogenesis in chronic vascular occlusion.

It is an object of the invention to overcome at least one of the above-referenced problems.

### Statements of Invention

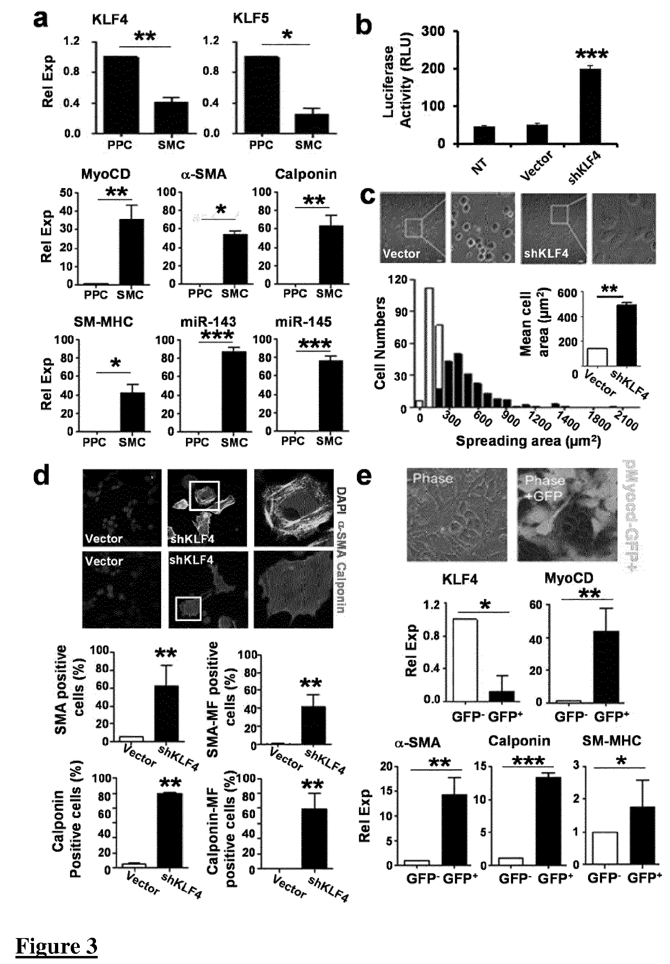

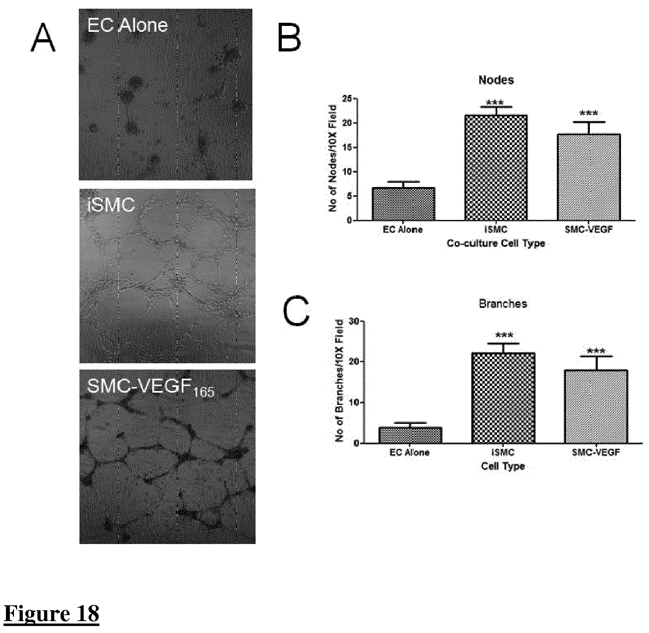

The invention is based on the finding that primitive progenitor cells (PPC) can be directly converted into induced smooth muscle-type cells (iSMC) by myocardin upregulation, typically using single transcription factor knockdown or transactivator overexpression (Figs 3, 4, 8, 9, 10 and 14). iSMC exhibit immuno- and cytoskeletal phenotype, calcium signalling profile and contractile responses similar to *bona fide* SMC and can be scaled-up sufficiently to replace abdominal aorta in an *in vivo* rat model. iSMC also exhibit an angiogenic potential that is superior to the VEGF-engineered SMC's described in Kumar *et al* (Fig. 18). Human iSMC can be derived from myocardin transactivator-manipulated PPC obtained from, for example, bypass filters of cardiac surgery patients or peripheral blood or bone marrow of healthy patients.

The invention also relates to the use of the iSMC's of the invention for *in-vivo* and *in-vitro* cell-based generation of tissue, ideally autologous cell-based generation of tissue. In one embodiment, the cells of the invention may be employed to treat vascular occlusion (e.g chronic vascular occlusion) in a mammal in which an intravascular cell delivery device (ICDD) comprising the cells of the invention is implanted in the vasculature of the patient at or close to the site of the occlusion (wherein the cells typically promote re-vascularisation or development of micro-vasculature) through development of an angiogenic network that connects the proximal vessel to the distal vessel across the occlusion.

In a first aspect, the invention provides an induced smooth muscle-type cell (iSMC) having a smooth muscle cell phenotype and prepared by reprogramming a primitive progenitor cell comprising introducing into the cell an agent capable of increasing the expression of myocardin in the primitive progenitor cell.

Typically, the agent is selected from a transcription factor knockdown agent (for example a KLF4 antagonist), an artificial transactivator protein (for example a myocardin promotor), or a nucleic acid construct comprising a myocardin transgene typically under the control of a transcriptional promotor.

The invention also relates to an induced smooth muscle-type cell (iSMC) that is derived from a primitive progenitor cell by myocardin transactivator modulation and exhibits a smooth muscle cell phenotype.

The invention also relates to a method for preparing an induced smooth muscle-type cell (iSMC) by reprogramming a primitive progenitor cell comprising introducing into the cell an agent capable of increasing the expression of myocardin in the primitive progenitor cell.

Typically, the iSMC exhibit an immunological response that is similar to smooth muscle cells. Typically, the iSMC exhibit a cytoskeletal phenotype that is similar to smooth muscle cells. Typically, the iSMC exhibit a contractile response that is similar to smooth muscle cells. Typically, the iSMC exhibit a calcium signalling profile that is similar to smooth muscle cells.

The invention also relates to a method of selecting for an induced smooth muscle-type cell from a culture of reprogrammed primitive progenitor cells comprising the step of selecting cells that express excess beta 1 integrin compared to the primitive progenitor cell from which they were reprogrammed and derived.

The invention also relates to an induced smooth muscle-type cell (iSMC) of the invention (or PPC cells) for use in cell based *in-vivo* and *in-vitro* tissue regeneration in a mammal.

The invention also relates to an induced smooth muscle-type cell (iSMC) of the invention (or PPC cells) for use in cell based *in-vivo* and *in-vitro* regeneration of the hollow organ tissue, especially hollow organ tissue selected from vasculature tissue, respiratory tract tissue, gastrointestinal tract tissue, and genito-urinary tract tissue.

The invention also relates to an induced smooth muscle-type cell (iSMC) according to the invention, for use in a method of treatment of chronic vascular occlusion in a mammal (typically by induced re-vascularisation), in which an intravascular cell delivery device (ICDD) comprising the iSMC's is implanted into the mammal, and wherein the ICDD typically comprises a hollow tubular structure with a multi-layered lattice configuration that can be constructed from a metal, a non-bioabsorbable polymer or a bioabsorbable polymer. In another embodiment the cell delivery device may constitute a strip of mesh material which can be attached onto a stent in whole or in part be delivered via a balloon or deployed on a flexible manoeverable wire that allows either the strip or the cylindrical mesh to be expanded from a compressed or constricted conformation. The intravascular cell delivery device comprising the induced smooth muscle-type cell is implanted into the vasculature at or close to a vascular occlusion. Typically, the cells of the invention promote vascularisation by sending out signals when they encounter the hypoxic environment of the blockage which stimulates the body to form new blood vessels around the blockage, thereby re-establishing downstream, oxygen supply.

The invention also relates to an induced smooth muscle-type cell (iSMC) according to the invention, for use in a method of promoting re-vascularisation or neovascularisation of a target site in a mammal, in which an intravascular cell delivery device comprising the induced smooth muscle-type cell is implanted into the vasculature at or close to the target site. Typically, the cells of the invention promote neovascularisation by sending out signals when they encounter the hypoxic environment of the blockage which stimulates the body to form new blood vessels around the blockage, thereby re-establishing downstream, oxygen supply.

The cell based tissue regeneration may be autologous cell based tissue regeneration.

The invention also relates to a bioactive tissue repair construct comprising a tissue repair construct seeded with induced smooth muscle-type cells (iSMC's) of the invention, PPC cells or with other angiogenic cells such as progenitor cells or mesenchymal stem cells that have the capacity to augment revascularizaion or neovascularization.

Typically, the tissue repair construct is selected from a scaffold containing a mesh structure derived from metals such as stainless steel, tantalum, or cobalt chromium. Alternatively the mesh structure may be manufactured from bioabsorable or non bioabsorbable polymers Suitably, the scaffold is an intravascular cell delivery device (ICDD) or a vascular graft. Examples of suitable ICDD's are described in US 6,913,762 and Biomaterials 35 (2014) 9012.

The invention also relates to a method for cell-based *in-vivo* repair, typically autologous cell-based *in-vivo* repair, of a defect site in mammalian tissue, for example hollow organ tissue such as a blood vessel or a respiratory, gastrointestinal, or genito-urinary tract, in a patient comprising the steps of implanting a bioactive tissue repair construct of the invention at the defect site in the patient.

The invention also relates to mammalian tissue generated *in-vitro* according to a method of the invention.

The invention also relates to a pharmaceutical formulation comprising induced smooth muscle-type cells of the invention and a pharmaceutically acceptable excipient.

### Brief Description of the Figures

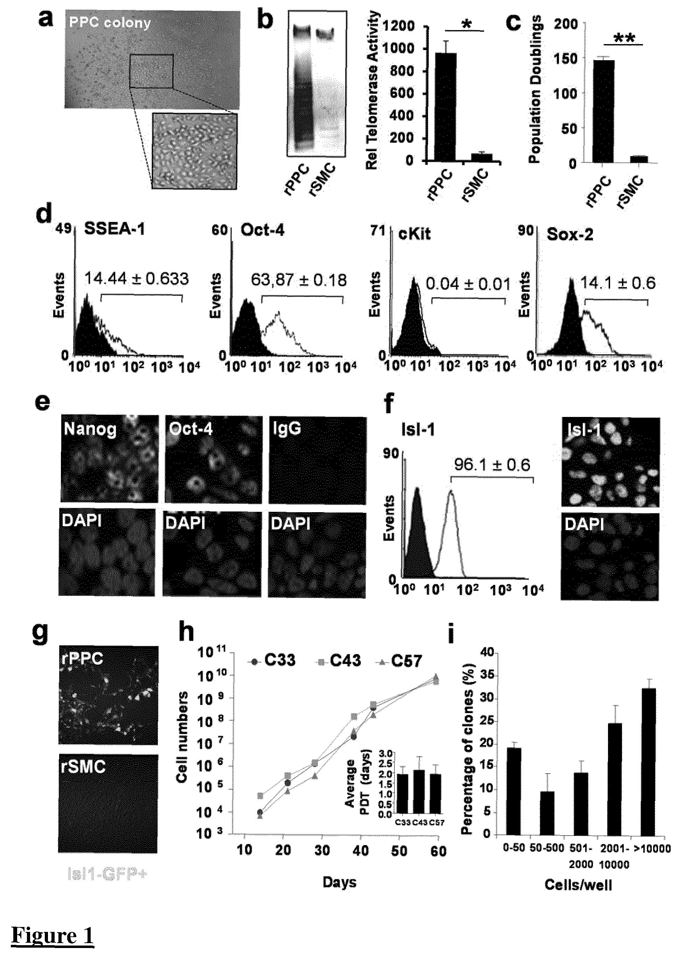

***Figure 1*** ***Rat PPC exhibit stem cell characteristics.* (a)** Phase contrast microscopy of rat PPC colony. **(b)** High telomerase activity in clonally expanded PPC relative to rat SMC (*p < 0.05). **(c)** Following initial isolation, PPC are capable of significantly more population doublings (>150; **P < 0.01) than SMC (20.6 ± 1.45). **(d)** Expression profiling of stem cell markers by flow cytometry shows that PPC coexpress SSEA-1, Oct-4 and Sox-2 but not cKit. **(e)** Immunostaining shows high levels of intracellular Oct-4 and Nanog in PPC. **(f)** PPC express significant levels of nuclear localizing Isl-1 and therefore **(g)** enrichment of PPC by transduction of a lentiviral vector encoding the Isl-1 promoter driving GFP prior to FACs sorting could be used to generate single cell-derived clones that display unlimited self-renewal, high telomerase activity and clonogenic capacity. **(h)** Population doubling time indicating the growth kinetics of three PPC clones with high proliferative potential (C33, C43 and C57). Insert: Mean doubling time in days of selected PPC clones. **(i)** Hierarchy of proliferative potential of PPC progeny derived from single cells plated in individual wells of a 96 well plate after 14 days of culture. All above data presented is obtained from 3 independent BM isolation experiments.

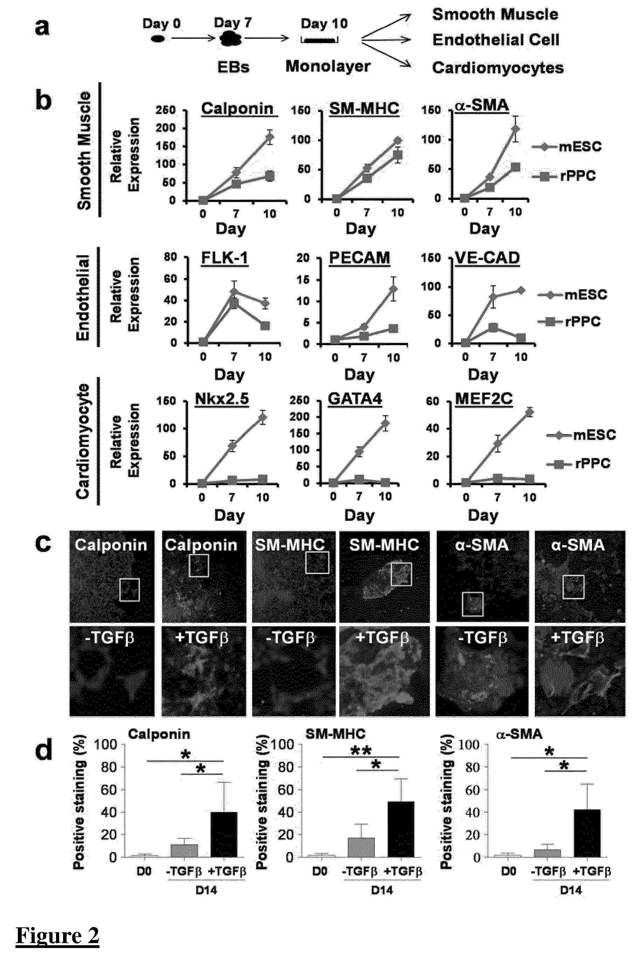

***Figure 2*** ***Rat PPC differentiation potential.* (a)** A schematic outlining the model for spontaneous smooth muscle, endothelial and cardiomyocyte lineage differentiation from PPC-derived embryoid bodies (EBs). The experimental design was based on widely used culture conditions for smooth muscle, endothelial and cardiomyocyte differentiation of mouse embryonic stem cells (mESC) post-EB formation. Rat PPC were cultured in equivalent conditions. **(b)** Quantitative RT-PCR analysis of smooth muscle marker (Calponin, SM-MHC and α-SMA), endothelial marker (FLK-1, PECAM and VE-Cad) and cardiac marker (Nkx2.5, GATA4 and MEF2C) expression levels at days 0, 7 and 10 following spontaneous differentiation from mESC- and PPC-derived EBs. **(c)** Immunostaining analysis of Calponin, SM-MHC and α-SMA following spontaneous differentiation, in the absence or presence of TGF 1, from PPC-derived EBs at day 14. **(d)** Quantification of immunostaining analysis by FACS (*p < 0.05, **p < 0.01).
***Figure 3*** ***KLF4 silencing promotes smooth muscle differentiation in PPC*. (a)** Quantitative RT-PCR characterization of PPC relative to SMC showing high expression of the smooth muscle differentiation repressors KLF4 and KLF5 in PPC and low expression of the hallmark smooth muscle differentiation markers myocardin, α-SMA, Calponin, SM-MHC, miR-143 and miR-145 (*p < 0.05, **p < 0.01, ***p < 0.001). **(b)** shKLF4-PPC transfected with a pGL3Enhancer vector encoding the myocardin promoter driving luciferase expression. Reduced KLF4 expression enhanced myocardin promoter-driven gene expression approximately 4-fold (***p < 0.001). (**c**) Phase contrast microscopy of vector control- or shKLF4-transduced PPC. Mean cell area of vector control or shKLF4-transduced PPC showed > 3-fold enlargement of cells in the latter group (**p < 0.01). (**d**) Analysis of α-SMA and Calponin expression and myofilament protein organisation in PPC after shKLF4 transduction in the presence of TGFβ1 throughout. Histograms below represent quantification of data (**p < 0.01). (**e**) shKLF4-PPC transduced with a lentiviral vector encoding the myocardin promoter driving GFP expression. GFP+ cells displayed reduced KLF4 expression but enhanced myocardin, α-SMA, Calponin and SM-MHC gene expression relative to GFP- cells (*p < 0.05; **p < 0.01, ***p < 0.001).

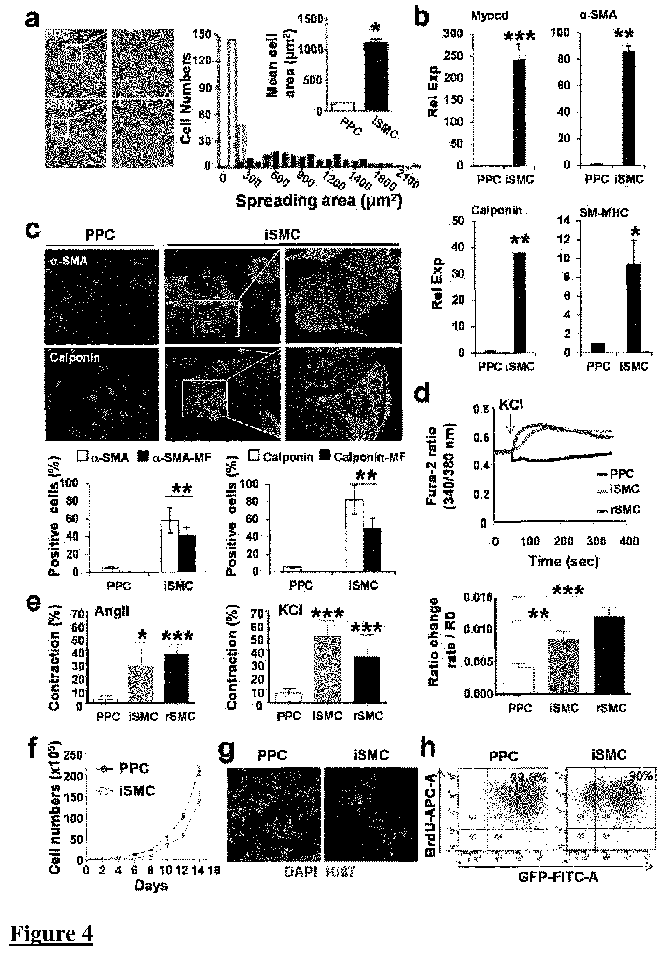

***Figure 4*** ***Myocardin overexpression generates induced smooth muscle cells (iSMC).*** (**a**) Phase contrast microscopy of PPC and iSMC. Mean cell area of PPC and iSMC shows an enlargement of cells in the latter group (*p < 0.05). (**b**) Quantitative RT-PCR analysis showing increased myocardin, α-SMA, Calponin and SM-MHC expression in iSMC when compared to PPC (*p < 0.05; **p < 0.01 and ***p < 0.001). (**c**) Immunostaining analysis of α-SMA and Calponin expression and myofilament organization in iSMC compared to PPC (**p < 0.01). (**d**) Ca²⁺ signalling in response to 60 mM KCl in iSMC was enhanced to levels similar to mature SMC (relative to control PPC; **p <0.01; ***p < 0.001). Histogram below represents the rate for 0.1 ratio increase over Ro, showing that iSMC and rSMC express more voltage-gated Ca²⁺ channels compared to PPC (**e**) Single cell contraction responses to 10 nM angiotensin II or 60 mM KCl in iSMC relative to PPC and rSMCs (*p < 0.05, ***p < 0.001). (**f-h**) No significant effect on cell proliferation after iSMC conversion from PPC. Myocardin/GFP⁺ iSMC were enriched by FACS sorting to >95% and growth rate compared to pure PPC culture. 10⁵ cells were seeded in 6-well plate at day 0 and counted every 2 days until day 14. Data presented is obtained from 3 independent experiments (**f**). Ki67 (**g**) and BrdU (**h**) staining were performed and analysed by confocal microscopy and FACS analysis.

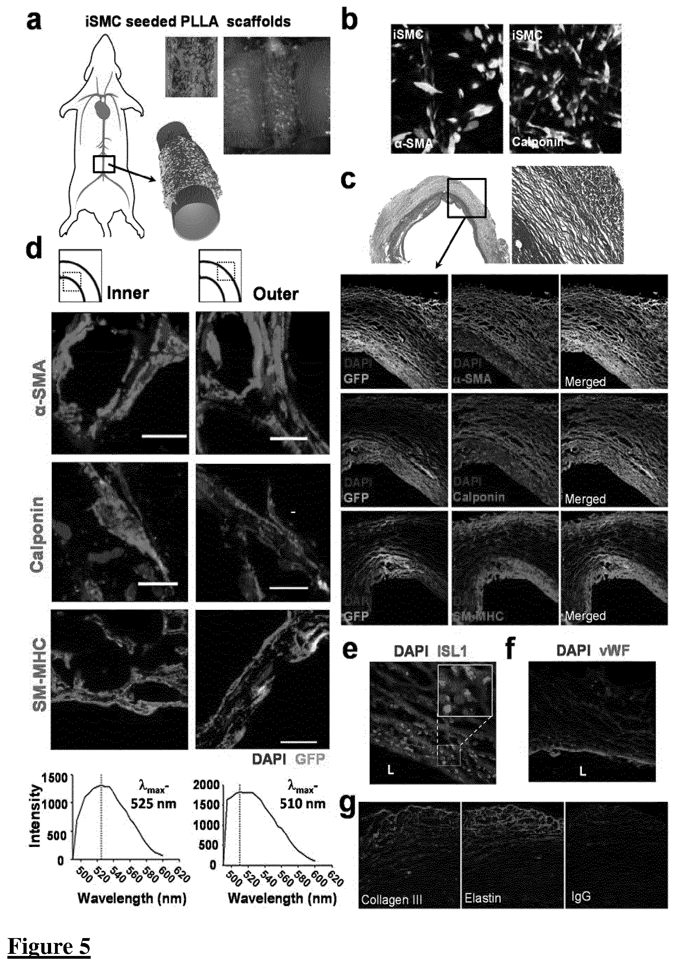

***Figure 5*** ***Evaluation of iSMC as a potential smooth muscle cell source for TEVG.*** (**a**) and (**b**) Myocardin/GFP⁺-iSMC were seeded in PLLA biodegradable tubular scaffolds (**a**) and seeding verified by fluorescence microscopy at low power magnification (**a** and **b**; green) and by *in situ* immunofluorecent staining of cells for α-SMA and calponin (**b**; red). (**c**) Surgical interpositional grafting of the iSMC-seeded PLLA tubular scaffold in the descending abdominal aorta below the renal artery branch points was performed in rats (*n* = 7). Two weeks following grafting, the TEVG was harvested for histological and immunofluorescent analysis of native GFP and smooth muscle marker staining. (**d**) Spectral confocal microscopy scanning showed GFP co-localized with SMC markers in both the inner and outer regions of the grafts, as indicated. Histograms below represent the mean GFP intensity emission spectra of the inner (left) and outer (right) regions with λₘₐₓ values of 525 and 510 nm respectively. (**e-g**) Isl-1 (**e**), vWF (**f**), collagen III and elastin (**g**) immunostaining of the inner and outer regions of the implanted vessel.

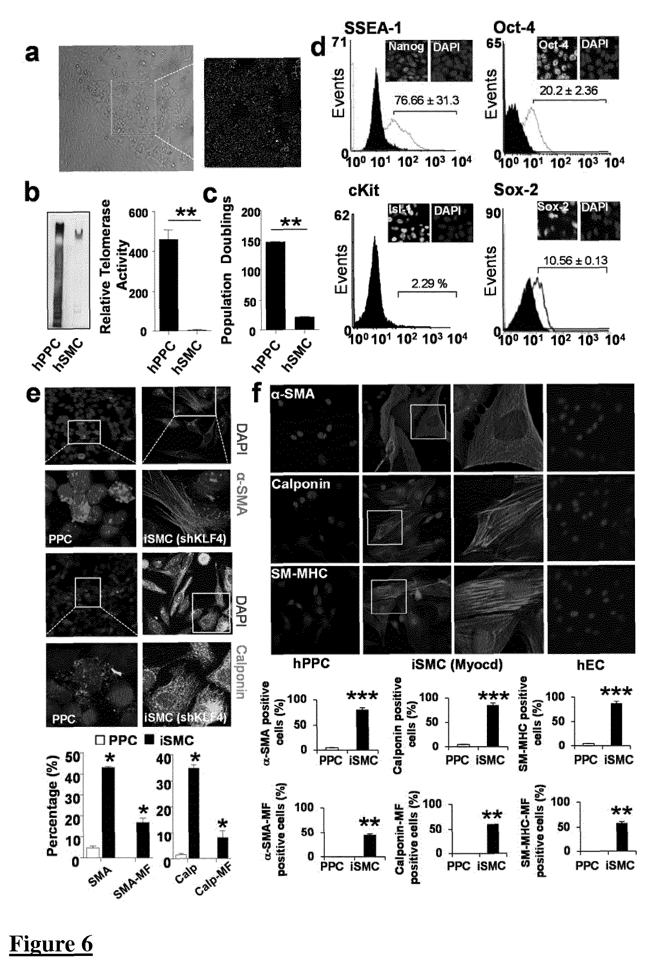

***Figure 6*** ***Human PPC possess similar phenotype and iSMC reprogramming potential.*** (**a**) Phase contrast microscopy of hPPC colony. (**b**) High telomerase activity in clonally expanded hPPC relative to hSMCs (**: p < 0.01). (c) hPPC are capable of significantly more population doublings (>150) than hSMCs (9.7 ± 1.2, **: p < 0.01). (**d**) Expression profiling of stem cell markers by flow cytometry shows that hPPC express SSEA-1, Oct-4 and Sox-2 but not cKit. Inserted panels: Immunofluorescence imaging of nuclear localizating Nanog, Oct-4, Isl-1 and Sox-2 in PPC. (**e**) Analysis of α-SMA and Calponin expression and myofilament organisation in iSMC (shKLF4), in the presence of TGFβ1, compared to hPPC (*p < 0.05). (**f**) Analysis of α-SMA, Calponin and SM-MHC expression and myofilament organisation in iSMC (Myocd) compared to hPPC alone (**p < 0.01, ***p < 0.001) and hEC transduced with Myocd as an extra cell control. In both cases (**e**-**f**) lower panels represent quantification of data. Data presented from 7 independent human filter isolation experiments (*n* = 7).

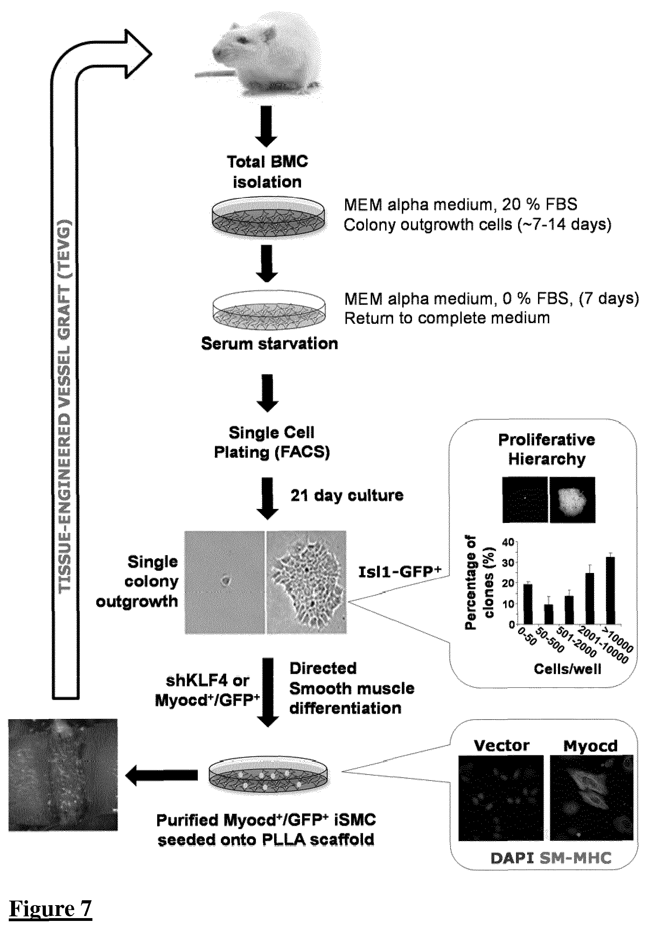

***Figure* 7****:** Outline schematic of PPC isolation from rodent bone marrow and generation of iSMC for use in tissue engineered vascular grafts *in vivo.*

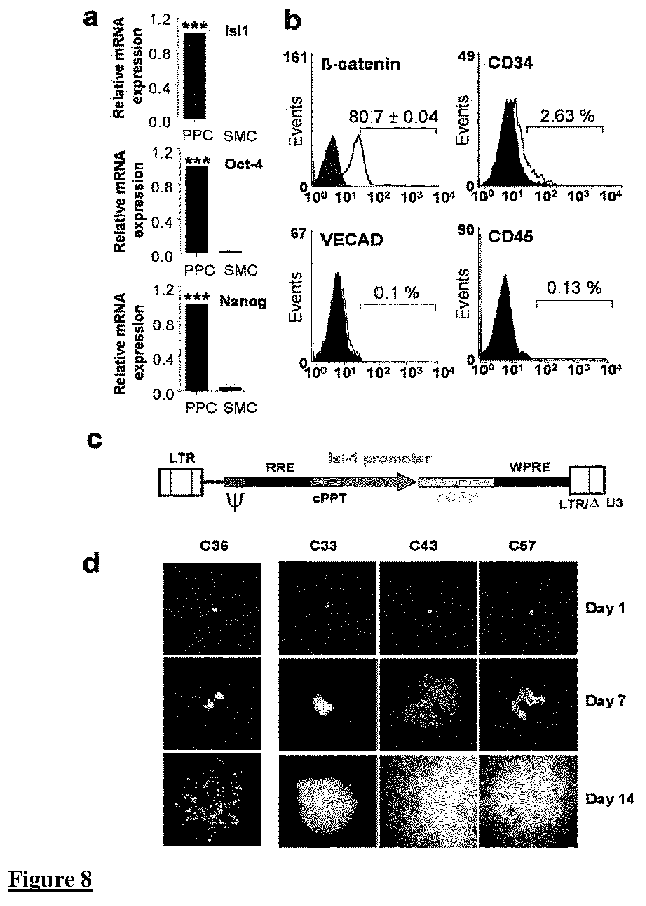

***Figure 8*** ***Rat PPC possess stem cell characteristics***. (**a**) Quantitative RT-PCR and (**b**) immunostaining analysis show high levels of Isl-1, Oct-4, β-catenin and Nanog are expressed in PPC but these cells do not express CD34, VE-cadherin (VECAD) and CD45 (***p < 0.001). (**c**) Schematic of lentiviral vector encoding Isl-1 promoter driving GFP expression. (**d**) Isl-1 expressing PPC progeny derived from single cells plated on individual wells at days 0, 7 and 14 of culture illustrating 3 high proliferative potential PPC colonies (C33, C43, C57).

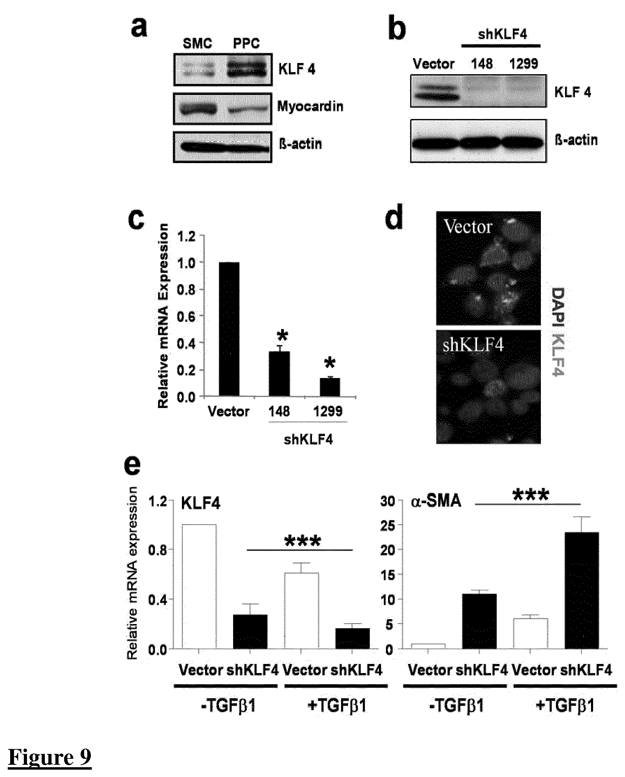

***Figure 9*** ***KLF4 silencing promotes smooth muscle differentiation of PPC*.** (**a**) Relative KLF4 and Myocardin protein levels in PPC and SMC with β-actin acting as a loading control. (**b**) Immunoblot analysis and (**c**) quantitative RT-PCR confirming the knockdown of KLF4 protein and mRNA levels, respectively, in PPC transduced with shKLF4 variants 148 and 1299 (*p < 0.005) compared to vector control. (**d**) Immunostaining of shKLF4-transduced PPC showing a reduction in KLF4 protein expression levels relative to control vector-transduced PPC. (**e**) Quantitative RT-PCR analysis of KLF4 and α-SMA mRNA levels in vector control- and shKLF4-transduced PPC in the presence and absence of TGFβ1 (***p < 0.001).

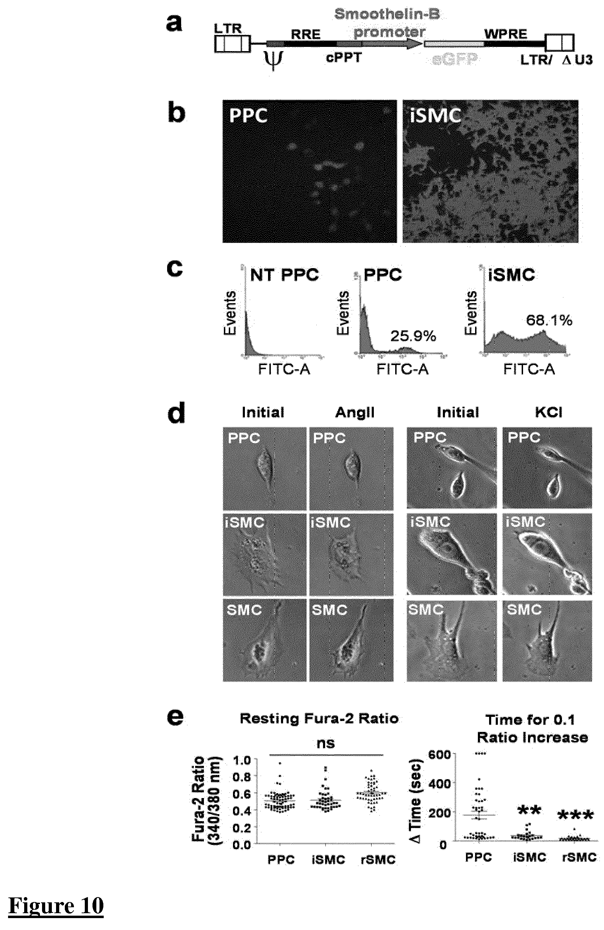

***Figure 10*** ***Myocardin overexpression in PPC geuerates mature functional iSMC**.* (**a**) Schematic of lentiviral vector encoding Smoothelin-B promoter driving GFP expression to assess mature SMC phenotype. (**b**) High Smoothelin-B promoter expression in iSMC in comparison to PPC, indicating mature differentiated SMC transcriptional activity. (c) FACS analysis quantification of data in **b** (NT indicates nontransduced PPC). (**d**) Representative images for single cell contraction responses to 10 nM angiotensin II or 60 mM KCl in iSMC relative to PPC and rSMC. Left panels are initial images; right panels are 15 minutes after stimulation. (**e**) Raw data for resting Fura-2 ratios (left) and Time for 0.1 ratio increases (right) in Ca²⁺ signalling assays using iSMC, PPC and rSMC showing that iSMC and rSMC express more voltage-gated Ca²⁺ channels in comparison to PPC (quantification of this data on Figure 4d).

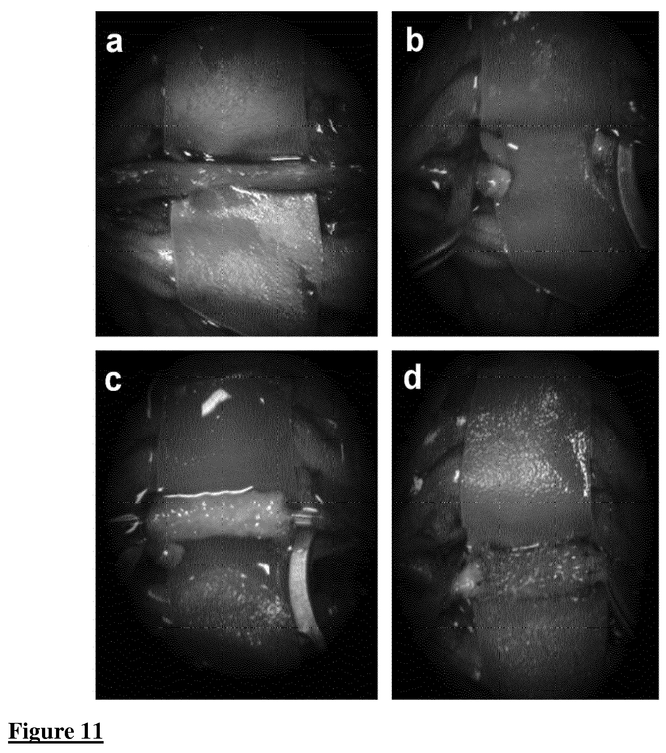

***Figure 11*** **Interpositional grafting of the myocardin/GFP⁺ iSMC-seeded PLLA scaffolds.** iSMC seeded scaffolds were placed at the infra-renal aortic position as an interposition graft with end to end anastomosis (*n* = 7). (**a**) demonstrates accessing the aorta separated from the inferior vena cava. (**b**) demonstrates the aorta after cross clamping and excision of a segment of aorta to allow for interposition of iSMC-PLLA scaffold (**c**). (**d**) End to end anastomosis was performed by microscopic surgery. Successful anastomosis was confirmed by evidence of blood flow distal to the anastomosis and lack of any blood leakage at the anastomosis sites.

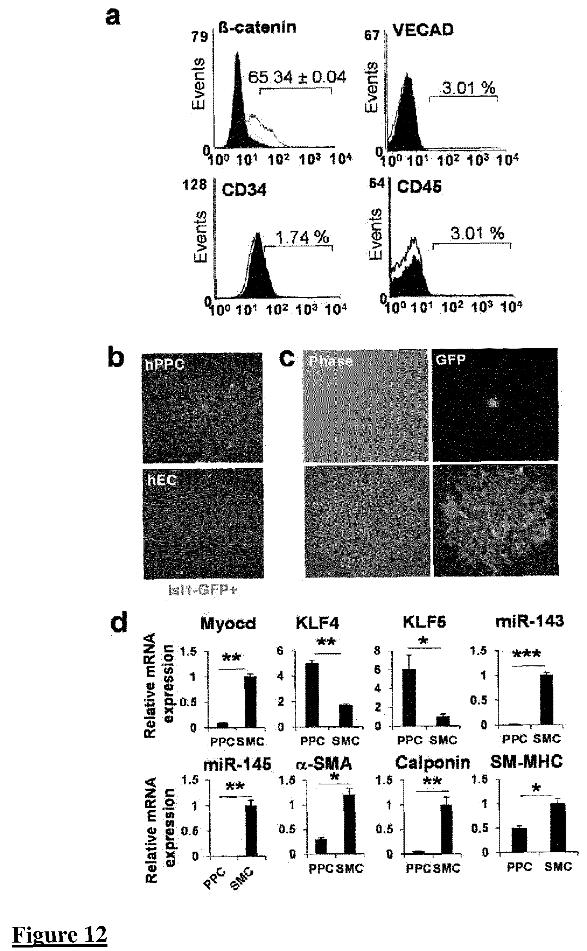

***Figure 12*** ***Human PPC possess stem cell characteristics***. (**a**) Expression profiling by flow cytometry show that hPPC express β-catenin but do not express CD34, VE-CAD and CD45. (**b**) Human PPC display high levels of Isl-1 promoter activity compared to human endothelial cells (hECs); therefore enrichment of hPPC by transduction of Isl-1 promoter driving GFP prior to FACS sorting can be used to generate single cell-derived clones that display unlimited self-renewal, high telomerase activity and clonogenic capacity (**c**). (**d**) Quantitative RT-PCR characterization of hPPC relative to human SMC showing low expression of smooth muscle differentiation marker myocardin, α-SMA, Calponin, miR-143 and miR-145 and high expression of smooth muscle differentiation repressors KLF4 and KLF5 (*p < 0.05, **p < 0.01, ***p < 0.001).

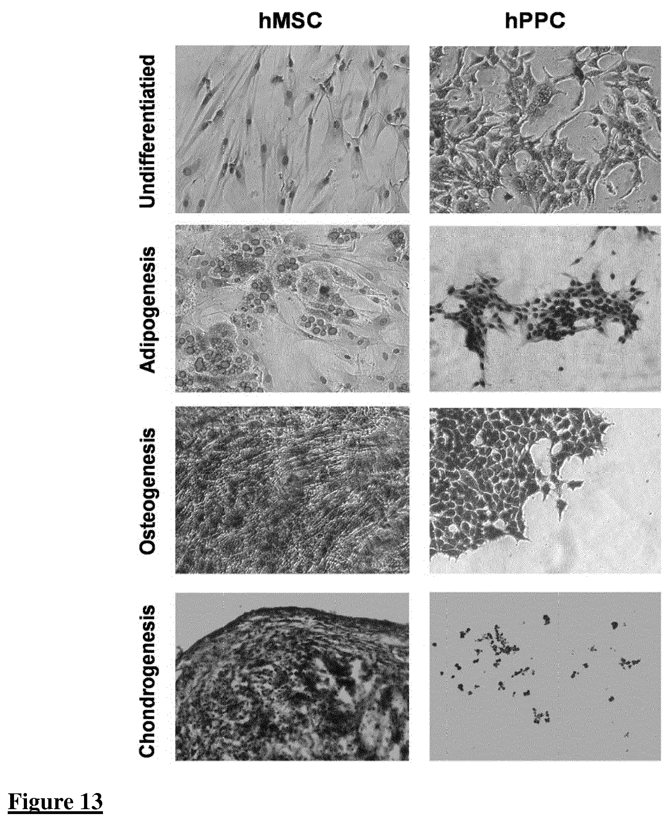

***Figure 13*** ***hPPC are not capable of mesenchymal differentiation**.* hPPC and human mesenchymal stem cells (hMSC) were cultured in basal media or in conditions favouring adipogenesis, osteogenesis or chondrogenesis with MSC staining positive for Oil Red O, alkaline phosphatase and toluidine blue respectively. In contrast, hPPC were negative in all three differentiation experiments.

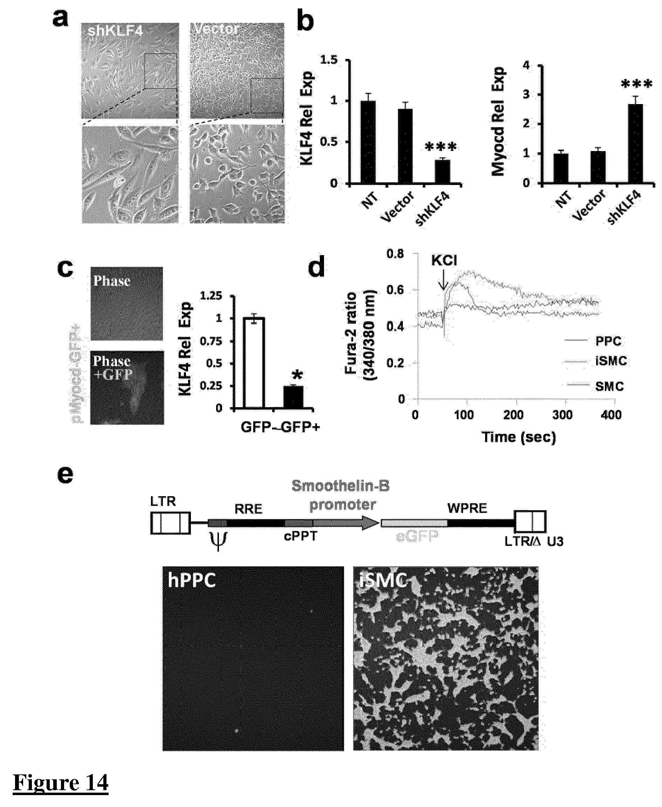

***Figure 14*** ***KLF4 silencing promotes smooth muscle cell differentiation and myocardin overexpression generates induced smooth muscle cells (iSMC)***. (**a**) Phase contrast microscopy of vector control- or shKLF4-transduced hPPC. (**b**) Quantitative RT-PCR analysis of KLF4 and myocardin mRNA levels in non-transfected (NT), vector control- or shKLF4-transduced hPPC (***p < 0.001). (**c**) PPC transduced with a lentiviral vector encoding the myocardin promoter driving GFP expression. Myocardin promoter-GFP+ cells displayed reduced KLF4 gene expression relative to GFP⁻ cells (*p < 0.05) indicating interaction between KLF4 and myocardin promoter activity. (**d**) Ca²⁺ signalling in iSMC was enhanced to levels similar to SMC (relative to control hPPC). E: Schematic of lentiviral vector encoding the Smoothelin-B promoter driving GFP expression. High Smoothelin-B promoter activity in iSMC indicating mature SMC transcriptional profile compared to hPPC.

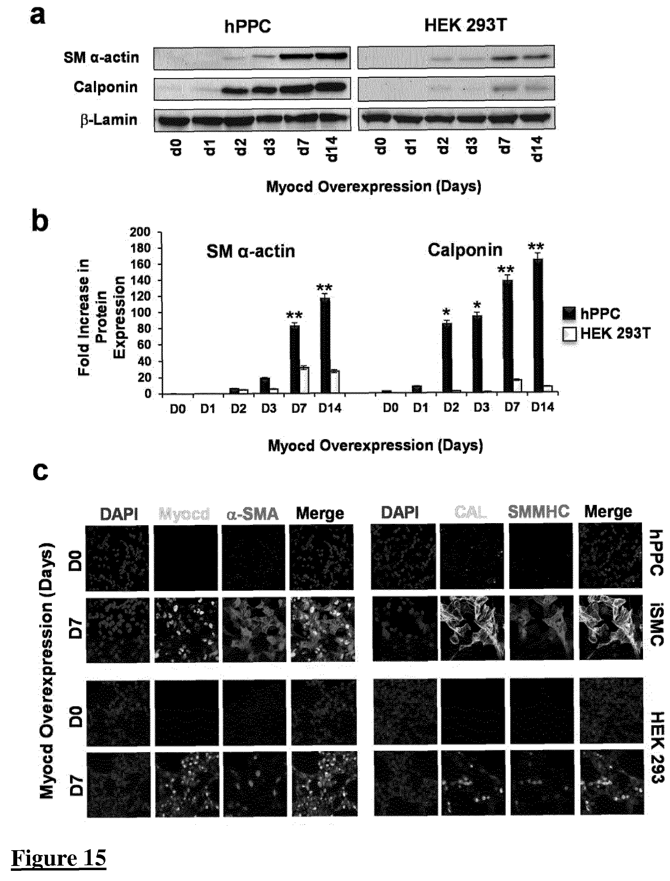

***Figure 15*** ***Overexpression of myocardin in hPPC induced smooth muscle marker protein expression in iSMC but not in HEK 293T cells***. (**a**) To determine whether forced expression of myocardin induces smooth muscle markers in hPPC and control HEK 293T cells, cells were transduced with LV_myocardin-HA and then were harvested over time (do, d1, d2, d3, d7 and d14) and analysed by Western blot analysis. Protein levels were normalised to β-Lamin protein expression. (**b**) Histograms below represent quantification of data (*p < 0.05, **p < 0.01). (**c**) Alternatively, the transduced cells were fixed at various time points (0 and 7 day post-transduction) for immunofluorescent staining with DAPI, *anti*-HA-tag (Myocd), *anti*-α-SMA, *anti*-Calponin and *anti*-SMMHC antibodies. Note marked co-labelling (merge images) of Myocardin and SM markers in iSMC but not transduced HEK 293 cells at day 7.

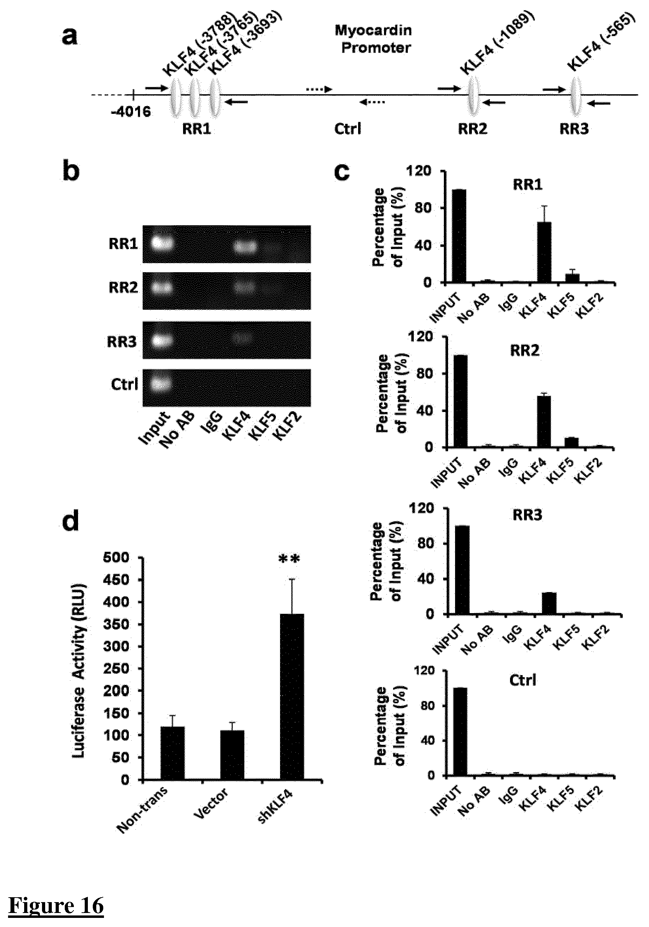

***Figure 16*** ***Chromatin immunoprecipitation (ChIP) analysis of the human myocardin gene promoter in hPPC***. (**a**) Schematic shows the forward and reverse primers used in ChIP assays to amplify either the RR1-3 (-3877 to -3586, -1221 to -962 and -789 to -433, respectively; solid line) or, as controls, a non-specific downstream region (Ctrl; -2226 to -1980; dashed line) of the human myocardin promoter. (**b**) Semi-quantitative and (**c**) quantitative RT-PCR ChIP analysis using cross-linked chromatin extracted from PPC where the images are representative of three independent experiments (*n* = 3). (**d**) shKLF4-hPPC transfected with a pGL3Enhancer vector encoding the myocardin promoter driving luciferase expression. Reduced KLF4 expression enhanced myocardin promoter-driven gene expression approximately 4-fold (**p < 0.01). Data was obtained from three independent experiments (*n* = 3).

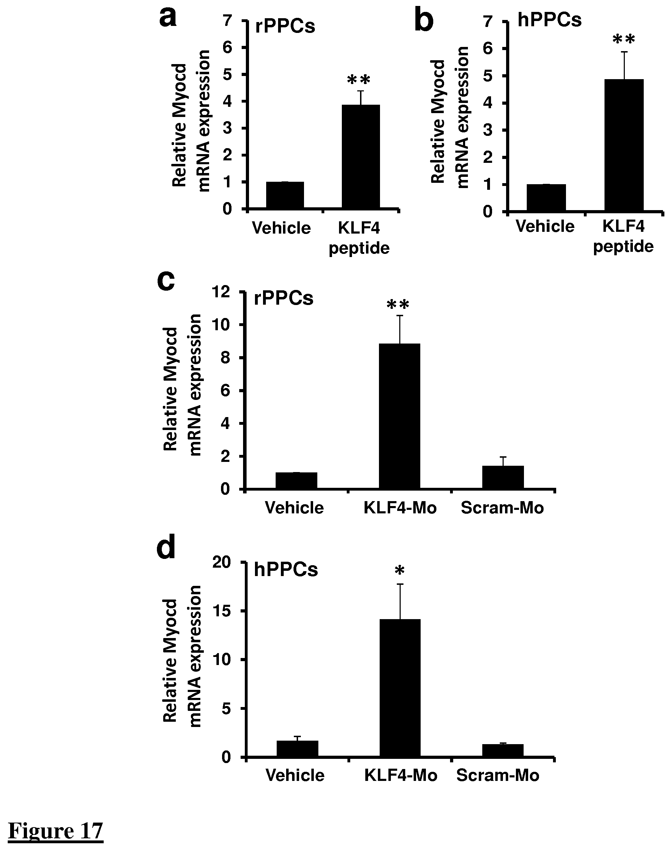

***Figure 17*** ***Non-viral KLF4 knockdown strategies markedly increase myocardin* mRNA *levels in hPPC***. (**a**-**b**) Quantitative RT-PCR analysis of myocardin mRNA levels in rPPC (**a**) and hPPC (**b**) treated with either vehicle or a KLF4 inhibiting peptide. (**c-d**) Quantitative RT-PCR analysis of myocardin mRNA levels in rPPC (**c**) and hPPC (**d**) transfected with KLF4 targeting morpholino oligos or their scrambled or inverted equivalents as controls(vehicle). Data was obtained from at least three independent experiments (*n* > 3; *p < 0.05, **p < 0.01).
***Figure 18******. Strong angiogenic potential of iSMC compared to genentically engineered SMC overexpressing VEGF (SMC-VEGF₁₆₅)***. Side panels show tubulation and angiogenic network formation of mixed culture of cells on Matrigel with greater number of nodes (right upper histograms) and branch points (right lower histograms) in iSMC co-culture with endothelial cells (EC) compared to SMC-VEGF or EC alone. This shows that iSMC are more angiogenic in vitro that previously published SMC-VEGF cells (Kumar et al Biomaterials 2014)

### Detailed Description of the Invention

### Definitions

"Primitive progenitor cell" or "PPC": means a self-renewing and clonogenic mammalian cell that can be obtained from peripheral blood or bone marrow of a mammal and expresses Isl-1 and exhibits preferential differentiation towards a smooth muscle cell fate rather than an endothelial or cardiomyocyte fate. Preferably, the PPC cells exhibit Oct-1. Preferably, the PPC express a smooth muscle cell marker. The PPC cells, and methods for isolating the cells, are described below and in International Patent Application No: PCT/IE2009/000041 (where they are referenced as "primitive vascular progenitor cells" or "vascular progenitor cells"). The present invention also relates to the PPC cells for use in a method of cell based, ideally autologous but may include allogeneic cell based, tissue regeneration in a mammal. The term PPC also includes derivatives of PPC's that genetically modified, for example genetically modified to express a transgene, for example a growth factor such as VEGF. Methods of engineering smooth muscle cells to express an exogenous gene are described in Kumar et al (Biomaterials 35 (2014) 9012-9022.

"Induced smooth muscle type cell" or "iSMC" means a mammalian cell that exhibits a smooth muscle cell phenotype and is derived from a primitive progenitor cell (PPC) by modulation of myocardin expression, typically by means of KLF4 inhibition or myocardin transactivator expression. The term iSMC also includes derivatives of iSMC's that are genetically modified, for example genetically modified to express a transgene, for example a growth factor such as VEGF. Methods of engineering smooth muscle cells to express an exogenous gene are described in Kumar et al..

"Smooth muscle cell phenotype": means that the cells when attached to a fibronectin as described below exhibit one or more, and typically all, of an increase in cell spreading area when attached to a fibronectin relative to PPC, augmentation of α-SMA/Calponin expression or augmentation of myofilament organisation when cultured in SmGM-2 medium with TGFβ1, and smooth muscle-associated gene expression changes (expression of smooth muscle markers α-SMA, Calponin, SM-MHC, and Smoothelin-B).

"An agent capable of increasing the expression of myocardin": means an agent that acts at a genetic level to increase myocardin expression within the cell. Typically, the agent is a nucleic acid construct but it may also be a protein or peptide (for example a peptide ligand specific to KLF4). Typically, the agent is selected from a transcription factor knockdown agent (for example a KLF4 antagonist), an artificial transactivator (modified RNA myocardin as recently described for a cytoprotective factor by CL Huang et al,Molecular Pharmaceutics 2015) or a nucleic acid construct comprising a myocardin transgene typically under the control of a transcriptional promotor. Methods for transducing mammalian cells with a myocardin gene are described in the literature (Van Tuyn, J. et al. Fibroblasts from human postmyocardial infarction scars acquire properties of cardiomyocytes after transduction with a recombinant myocardin gene. FASEB Journal: official publication of the Federation of American Societies for Experimental Biology 21, 3369-3379 (2007)). In one embodiment the agent is configured to promote expression of an endogenous myocardin gene, for example a myocardin-specific transactivator. In another embodiment, the agent comprises a myocardin transgene typically operatively connected to transcriptional promotor, ideally an inducible or constitutive promotor. In another embodiment the agent comprises a myocardin modified RNA that allows short term protein expression in the target cell. In another embodiment, the agent comprises an antagonist of a myocardin transcriptional repressor, typically a KLF4 antagonist. Typically, the agent is a nucleic acid construct comprising a KLF4 antagonist. This antagonist may be synthetic or a morpholino. Suitably, the agent is provided in the form of a vector suitable for use in gene therapy and comprising or encoding an agent capable of increasing the expression of myocardin. Suitably the vector is a viral vector, typically a lentivirus or an adeno-associated virus (AAV), preferably AAV virus serotype 2, although other AAV serotypes and other types of vectors may be employed such as for example other viral vectors, non-viral vectors, naked DNA and other vectors, examples of which will be apparent to a person skilled in the art. Typically, the agent is expressed singly from the vector (single delivery vehicle). In another embodiment, the agent is expressed together with another gene either from the single delivery vehicle or using two delivery vechicles, for example, a reporter gene.

"Increasing the expression of myocardin in the primitive progenitor cell": means increasing the expression of myocardin relative an untreated primitive progenitor cell.

"KLF4 antagonist": refers to a compound that is capable of decreasing the activity of KLF4 *in vivo.* The activity may be decreased in a number of different ways which will be apparent to a person skilled in the art, including reducing the expression of the protein (for example by means of low molecular weight inhibitors such as for example siRNA or shRNA), or by directly inhibiting the activity of the protein by administering a KLF4 inhibitor or an antibody that has specific binding affinity for KLF4. In a preferred embodiment of the invention, the invention relates to a low molecular weight inhibitor of KLF4, the details of which will be well known to the person skilled in the field of molecular biology, and which include siRNA, shRNA, miRNA, antisense oligonucleotides, and ribozyme molecules. Small inhibitory RNA (siRNA) are small double stranded RNA molecules which induce the degradation of mRNAs. Micro RNA's (miRNAs) are single stranded (~22nt) non-coding RNAs (ncRNAs) that regulate gene expression at the level of translation. Alternatively, small hairpin RNA (shRNA) molecules are short RNA molecules having a small hairpin loop in their tertiary structure that may be employed to silence genes. The design of miRNA or shRNA molecules capable of silencing KLF4 will be apparent to those skilled in the field of miRNA or shRNA molecule design. As an alternative, the activity of KLF4 can be modulated using antisense or ribozyme approaches to inhibit or prevent translation of KLF4 mRNA transcripts or triple helix approaches to inhibit transcription of the KLF4 gene. Antisense approaches involve the design of oligonucleotides (either DNA or RNA) that are complementary to KLF4 mRNA. The antisense oligonucleotides will bind to the complementary mRNA transcripts and prevent translation. Ribozyme molecules designed to catalytically cleave KLF4 mRNA transcripts can also be used to prevent translation and expression of RPT4. (See, e. g. , PCT International Publication WO90/11364, published October 4,1990 ; Sarver et al. , 1990, Science 247: 1222-1225). In one embodiment of the invention, the KLF4 inhibitor is an anti-KLF4 antibody (i.e. an antibody which specifically binds to human KLF4 protein). An example of such an antibody is sold by Abcam under the catalogue number ab151733. KLF4-specific antibodies may be produced using methods which are generally known in the art. In particular, purified KLF4 may be used to produce antibodies or to screen libraries of pharmaceutical agents to identify those which specifically bind KLF4. Antibodies to KLF4 may also be generated using methods that are well known in the art. Such antibodies may include, but are not limited to, polyclonal, monoclonal, chimeric, and single chain antibodies, Fab fragments, and fragments produced by a Fab expression library. Neutralizing antibodies (i.e., those which inhibit dimer formation) are generally preferred for therapeutic use. Single chain antibodies (e.g., from camels or llamas) may be potent enzyme inhibitors and may have advantages in the design of peptide mimetics, and in the development of immuno-adsorbents and biosensors (Muyldermans, S. (2001) J. Biotechnol. 74:277-302). For the production of antibodies, various hosts including goats, rabbits, rats, mice, camels, dromedaries, llamas, humans, and others may be immunized by injection with KLF4 or with any fragment or oligopeptide thereof which has immunogenic properties (especially the fragment specified above). Depending on the host species, various adjuvants may be used to increase immunological response. Such adjuvants include, but are not limited to, Freund's, mineral gels such as aluminum hydroxide, and surface active substances such as lysolecithin, pluronic polyols.

"Artificial myocardin-specific transactivator": means an agent designed to bind to a myocardin promotor and increase expression of the myocardin gene, or a nucleic acid encoding a transactivator protein. Examples of artificial transactivators suitable for the present invention include microRNA145 that will upregulate myocardin expression in target cell or KLF4 antagonists that will inhibit repression of myocardin gene expression.

"Myocardin transactivator modulation": means introducing into the cell an agent that increases the activity of a myocardin-specific transactivator protein.

"Immunological profile that is similar to smooth muscle cells": means intracellular antigenic profile that includes expression of myosin heavy chain and smoothelin as well as calponin and alpha smooth muscle actin.

"Cytoskeletal phenotype that is similar to smooth muscle cells": means high myofilament content co expressing smooth muscle specific markers such as myosin heavy chain and calponin.

"Contractile response that is similar to smooth muscle cells": means similar contractile response to smooth muscle specific agonist such as angiotensin 2 and bradykinin.

"Calcium signalling profile that is similar to smooth muscle cells": means calcium transients in response to ratiometric calcium indicator such as Fura 2 acetomethyl ester that is measured by epifluorescent videomicroscopy .

"Tissue repair construct" means a three dimensional construct (scaffold) having a solid or semisolid 3-D matrix capable of being seeded or coated with cells and is typically dimensioned for use in *in-vivo* repair of mammalian tissue, for example repair of vasculature defects. A "bioactive tissue repair construct" means a tissue repair construct that comprises induced smooth muscle cells of the invention (i.e. seeded or coated with cells). Examples of suitable constructs include solid scaffolds, frameworks, stents, intravascular cell delivery devices, vascular grafts and hydrogels. Ideally, the construct is percutaneously deliverable. Examples of tissue repair constructs for use with the present invention are described below and in Kurobe et al (Stem Cell Transl Med. 2012 Jul; 1(7):566-571), Kumar et al (Biomaterials 35 (2014) 9012-9022), and US 6,913,762, the relevant details of which are incorporated herein by reference.

"Intravascular cell delivery device" or "ICDD") means a device that is configured to carry cells, be implanted in the vasculature (for example, the coronary arteries), and release the cells *in-situ* within the vasculature. Typically, the device is configured for percutaneous delivery. Examples of suitable ICDD's include stents, for example the stents disclosed in Kumar et al (Biomaterials 35 (2014) 9012-9022), and US 6,913,762, the relevant details of which are incorporated herein by reference. Other ICDD's include hollow tubular structures, optionally formed of a multi-layer lattice configuration, or strips of mesh material. The ICDD may be expandable, preferably, self-expandable, from a contracted or constrained configuration to an expanded (deployed) configuration. The ICDD may be formed of a metal, a polymer, or a combination of metal and polymer. The polymer may be bioabsorbable or non-bioabsorbable.

"Seeding" or "coating" as applied to a tissue repair construct should be understood to mean incorporating a biological material into or onto the tissue repair construct. Method of seeding a construct include soaking the construct in a solution containing the biological material for a sufficient time to allow the biological material infiltrate the pores of the construct or adhering cells to a coating on the construct. Method of coating a construct with cells include applying a natural or synthetic porous biodegradable biocompatible coating to the construct, and seeding the coating with cells. Examples of suitable coatings include collagen and PLGA.

"Patient" means a mammal, typically a human, and typically a human with a tissue defect, for example a defect, disease, condition or damage in vasculature tissue or respiratory tract tissue, genito-urinary tract tissue, gastrointestinal tract tissue.

"Autologous": as applied to the therapeutic methods and uses of the invention means treatment of a patient with iSMC's that are generated using PPC derived from the same patient.

### Experimental

### Materials

Dual Luciferase® Reporter Assay System, pRL-Thymidine Kinase (pRL-TK), Lipofectamine 2000, TRIzol reagent, and SuperScript® III reverse transcriptase were from Invitrogen. Antibodies *anti*-KLF4, *anti*-KLF5, *anti*-KLF2, *anti*-CD34, *anti*-CD45, *anti*-cKit, *anti*-VE-Cadherin, normal rabbit IgG, goat *anti*-rabbit horseradish peroxidase and goat *anti*-mouse HRP were all from Santa Cruz. TGFβ1 and the *anti*-myocardin antibody were from R&D systems. Albumin from bovine serum (BSA), DMEM, Medium 199 (M199), 4',6-diamidino-2-phenylindole (DAPI), and *anti*-β-actin antibody were from Sigma. The *anti*-vWF antibody and anti-α-SMA antibody were from DAKO. Antibodies against Ki67, collagen, elastin, Oct-4, Nanog, Islet-1 (Isl-1), and smooth muscle-myosin heavy chain 11 (SM-MHC) were from Abcam. *Anti*-Calponin antibody was from Insight Biotechnology Limited. *Anti*-SSEA-1 and control IgG antibodies were from BD Biosciences. *Anti*-Isl-1 (39.4D5) was from DSHB. Fetal bovine serum (FBS), L-glutamin, penicillin/streptomycin, NEAA, and sodium pyruvate were from Gibco. MEM alpha medium was from PAA laboratories. KLF4 peptide (NBP1-77382PEP) was from Novus Biologicals. Morpholino oligos were from GeneTools. EGM-2 and SmGM-2 medium were from Lonza. PLLA BIOFELT nonwoven scaffold was from Biomedical Structures. DNA oligos were from Eurofins MWG Operon.

### Animals

All *in vivo* procedures on animals were conducted in accordance with University College Cork Animal Use Ethics Committee standards. Fischer rats were obtained from breeding colonies in Biological Service Unit of University College Cork and originally sourced from Charles River Laboratories, UK. Rat used in experiments were 12-18 weeks of age weighing 150-400 g.

### Human subjects

The Pall LeukoGuard® LGB arterial line leukocyte reduction filters with bypass Loop for coronary bypass (Pall Corporation, Ireland) were collected from cardiopulmonary bypass machine in the cardiac surgery operating theatres of Cork University Hospital. Coronary bypass filter collection and *in vitro* processing of the filters was carried out under human ethical guidelines of University College Cork and Cork University Hospital. Human subjects without current evidence of infection, myocardial infarction in the previous month, with diabetes mellitus, with previous CABG history, currently taking sotalol, flecainide or amiodarone, active malignancy and known chronic inflammatory or auto-immune disease were excluded from the study.

### Cell culture

Human aortic smooth muscle cells (hAoSMC) and rat smooth muscle cells (rSMC) were obtained from Lonza, Basal, Switzerland and routinely cultured in SmGM-2 medium. Murine ESCs E14tg2a (ATCC) were cultured in MEM alpha medium supplemented with 15% FBS, 1% L-glutamine, penicillin/streptomycin, NEAA and sodium pyruvate supplemented with 1,000 U/ml ESGRO (Chemicon, Millipore). HEK293T cells were cultured in DMEM medium supplemented with 10% FBS. Human endothelial cells (hEC) were obtained from Lonza, Basal, Switzerland and routinely cultured in M199 medium, 20% FBS and supplemented with H/ECGS (Promocell).

### Isolation and culture of stem cell-like primitive progenitor cells (PPC)

Rat bone marrow and peripheral blood from human leukocyte filters used in cardiopulmonary bypass procedures were used as sources of cells for rat and human PPC outgrowth, respectively. In brief, for the isolation of rat PPC (rPPC), bone marrow cells (BMC) were collected from Fisher rats by flushing femurs and tibias followed by lysis of remaining red blood cells. BMC were cultured at 10⁷ cells per well of a 6-well plate in MEM alpha medium supplemented with 20% FBS. Serum starvation induced stem cell outgrowth from rat bone marrow was performed according to a protocol described by Sauerzweig *et al.* with slight modifications (Sauerzweig, S., Munsch, T., Lessmann, V., Reymann, K.G. & Braun, H. A population of serum deprivation-induced bone marrow stem cells (SD-BMSC) expresses marker typical for embryonic and neural stem cells. Experimental cell research 315, 50-66 (2009)). Briefly, after the attached BMCs were cultured until forming a confluent cell layer the medium was exchanged by MEM alpha medium without FBS. BMCs were further grown 7 days in serum-free medium to allow the serum deprivation induced stem cell formation. Cell colonies appeared at 2-3 weeks after exchanging medium to normal condition.

For the isolation of human PPC (hPPC), cardiopulmonary bypass filters (Leukocyte filters) were obtained from Cork University Hospital under sterile conditions on ice. The two ends of the filter were clamped with sterile surgical clamps to prevent microbial contamination from the open ends. The membrane of filter was flushed with phosphate buffer saline (PBS; 150 ml) supplemented with penicillin/streptomyocin (100 U) and amphotericin B (250 ng/mL), and carefully layered on 15 ml Biocoll (Biochrome^{AG}). Biocoll density gradient centrifugation was performed at 1,100 g for 20 minutes at room temperature with slow acceleration and without break. A layer of mononuclear cells (MNC) was carefully collected post-density gradient centrifugation. The MNC were re-suspended in ACK Lysis Buffer (0.15 M NH₄Cl, 1 mM KHCO₃, 0.1 mM Na₂EDTA) to lyse the left over red blood cells (RBC). After RBC lysis, MNC were recovered by washing three times in MCDB131 media supplemented with hydrocortisone acetate (1 µg/ml), dibutyrl cAMP (0.5 mM), amphotericin B (0.25 µg/ml), VEGF (10 ng/ml), heparin (6.7 U/ml), penicillin/streptomycin (100 U/ml) and L-glutamine (1.6 mM), followed by centrifugation at 600 g for 5 minutes at 4°C. Cells were then seeded (1 x 10⁶ cells per well) in 6-well collagen-coated plate in EGM-2 medium supplemented with 10% FBS. Media was changed on day 2 to discard non-adherent cells and then every 3 days thereafter. PPC colonies appeared at day 21-30. Colonies were isolated by gentle pipetting with PBS and transferred to a new 24-well plate for outgrowth.

### Clonal colony formation and hierarchy of proliferative potential analysis

Single cell/clone selection was performed as previously described Metharom, P. et al. (Myeloid lineage of high proliferative potential human smooth muscle outgrowth cells circulating in blood and vasculogenic smooth muscle-like cells in vivo. Atherosclerosis 198, 29-38 (2008))with minor modifications. Clones were initially expanded from single cells using a limiting dilution method. For proliferative hierarchy analysis Isl1-GFP tracking was used. In brief, Isl1-GFP+ transduced single cells were identified by positive immunofluorescence and seeded in 96 well format (1 cell per well) using FACS sorting (BD FACSAria II, Becton Dickinson, Erembodgem, Belgium. After 2 weeks each well was examined for colony growth. Colonies were graded after 14 days in culture according to size into various categories: 0-50, 51-500, 501-2000, 2001-10,000 and > 10,001. The colonies with over 10,000 cells were expanded into 24 well plate and further expanded for long term culture.

### Telomerase activity

Telomerase activity was measured using TeloTAGGG Telomerase PCR ELISA (Roche Diagnostics) according to the manufacturer's instructions. PCR sample (20 µl) was separated on 12% polyacrylamide gel, transferred to a nylon membrane and probed with strepavidin-HRP for TRAP visualization. The images were quantified by densitometry using ImageJ software (NIH Bethesda, MD).

### Embryoid body (EB) formation and differentiation

To initiate mESC and rPPC spontaneous differentiation, cells were trypsinized and resuspended in MEM alpha medium (20% FBS, 1% penicillin/streptomycin, L-glutamine, NEAA and sodium pyruvate) at a concentration of 25,000 cells/ml. 20 1 hanging drops were made with the cell suspension and cultured for 2 days allowing the EB formation. EBs were then collected and grown in suspension. At day 7, EBs were plated onto gelatin-coated glass wells (2 to 3 EBs/well in 8-well glass slide, Millipore) in 400 µl of medium for further immunostaining. Around 20 EBs were collected for RNA isolation at day 7 and day 10. For directed SMC differentiation of rPPC, at day 7 EBs were plated onto fibronectin-coated glass wells and the media was exchanged to SmGM-2 and further supplemented with 5 ng/ml TGFβ1.

### Immunofluoreseent staining and flow cytometry

Cells were fixed in cold methanol for 20 minutes at 4°C and washed twice with PBS. Cells were blocked and permeablized with 0.1% Triton-X 100/PBS (0.2% for nuclear protein staining) and 3% BSA, stained with primary antibodies for 1.5 hours at room temperature or overnight at 4 °C, then visualized with secondary fluorescent conjugated antibodies (Invitrogen Molecular Probes). Nuclei were stained with DAPI. Immunofluorescent and native GFP images were captured using Nikon EZC1-3.30 software on a confocal microscope system (Nikon eC 1 plus, TE2000E). Primary antibodies used for this study are listed in Supplementary Table 1 along with their appropriate IgG isotype controls.

For extracellular antigens, cells were resuspended in 100 µl diluted primary antibody and incubated for 1 hour on ice. After washing with 1 mL of PBS + 0.5% BSA (PBSA), 100 µl diluted secondary antibody was added and incubated for 30 min on ice. Cells were washed with 1 ml PBSA and resuspended in 200-500 µl 1% paraformaldehyde for flow cytometric analysis.

### Plasmid construction and lentiviral vector production

For the construction of Isl1 promoter-GFP plasmid, DNA fragment upstream of the Isl1 start codon (-4681, +272) was amplified with KOD Hot Start Polymerase (Novagen, Madison WI) from BAC clone. PCR primers were forward (5'-GATGGTACCCTCAACTAAATGAGGCTAC-3') (SEQ ID 1) and reverse (5'-ATTGTCGACTTGTAAGAGGGAGTAATGTC-3' (SEQ ID 2). The PCR fragment was cloned into pCR2.1-TOPO (Invitrogen) and sequenced. pHR-CSGW was digested with EcoRI and BamHI to remove the SFFV promoter, and the Isl-1 promoter was cloned into the EcoRI-BamHI to create pHR-CIsl1GW. For the construction of the Smoothelin-B promoter-GPF plasmid, a 3561 bp human Smoothelin-B promoter region (-3372, +120) was amplified using high fidelity PCR (Roche Applied Science) and cloned into the EcoRI-BamHI sites of pHR-CSGW to generate pHR-SmthB. The Myocardin promoter reporter constructs were generated by PCR amplification of DNA fragments from genomic DNAs using the following primer set: forward 5'- AAAGACCTGAATTCTATCAATAACC-3' (SEQ ID 3); and reverse 5'-ACAACCCAGGATCCATCCAACTCTCCGTGA -3' (SEQ ID 4)
and cloned into pCR2.1-TOPO and sequenced. The myocardin promoter was then subcloned into either the GFP reporter plasmid pHR-CSGW (EcoRI to BamHI) to generate pHR-pMyocd or the luciferase reporter plasmid pGL3Enhancer (BglII to BglII) to generate pGL3E-pMyocd. For the construction of myocardin/GFP expression plasmid, the corresponding myocardin cDNA was obtained by PCR using the primer sets:
forward 5'- AAGCTAGCTAGCATGACACTCCTGGGGTCTGA-3' (SEQ ID 5) and reverse 5'-TATTCTTACGCCGGCGTCTAGAGTCTGCTGTACATT-3' (SEQ ID 6).

The PCR fragment was cloned into the pCR2.1-TOPO and sequenced, and subcloned into NheI-NotI site of pCDH vector (System Biosciences). The pKLO.1-puro lentiviral vector encoding a shRNA targeting KLF4 (shKLF4_B; target sequence GGACGGCTGTGGATGGAAA (SEQ ID 7)) was from Sigma. Full length human myocardin cDNA (pLV-CMV-Myocd-HA) was the generous gift of Dr D. E. Atsma from Leiden University Medical Center, the Netherlands (Van Tuyn, J. et al. Fibroblasts from human postmyocardial infarction scars acquire properties of cardiomyocytes after transduction with a recombinant myocardin gene. FASEB journal : official publication of the Federation of American Societies for Experimental Biology 21, 3369-3379 (2007).. pHR-CSGW vector was a kind gift from Dr Y Ikeda from Mayo Clinic , USA. Lentiviral vectors encoding GFP under the control of a human SFFV promoter were generated by a three-plasmid calcium phosphate transfection procedure. Briefly, HEK 293T cells were transfected with the transfer plasmid vector together with a packaging plasmid pCMV-ΔR8.91 and an envelope expression plasmid pMD.G. Viral supernatants were collected starting 48 hours and continuing for 4 consecutive days after transfection, and then filtered through a 0.45-µm filter. Viral supernatants were concentrated ~ 100-fold by ultracentrifugation in a Sorval centrifuge, for 90 min at 72,000 x g. Using these protocols, titers of ~5 x 10⁷ to 3 x 10⁸ TU/mL were achieved.

### Luciferase-based genetic reporter assays

PPC were co-transfected with various pGL3E-recombinant plasmids, encoding firefly luciferase, along with pRL-TK, encoding renilla luciferase, using FuGENE® HD (Promega) transfection reagent according to the manufacturer's instructions. Firefly and renilla luciferase activity was assayed after 48 h using the Dual Luciferase Assay System® (Promega). Relative firefly to renilla luciferase activities (arbitrary units) were calculated as a ratio and were expressed in relative luciferase units (RLU).

### Quantitative RT-PCR

Total RNA was isolated using TRIzol reagent according to the manufacturer's instructions (Invitrogen). cDNA was synthesised using SuperScript® III First-Strand Synthesis system (Invitrogen). QRT-PCR mixtures were prepared with LightCycler® 480 SYBR Green PCR Master Mix (Roche). Amplification was performed using a 384-microwell plate-based LightCycler® 480 Real-Time PCR System (Roche). Obtained values were normalized to glyceraldehyde 3-phosphate dehydrogenase (GAPDH). The relative expression was calculated according to the 2(-Delta Delta C(T)) method (Livak, K.J. & Schmittgen, T.D. Analysis of relative gene expression data using real-time quantitative PCR and the 2(-Delta Delta C(T)) Method. Methods 25, 402-408 (2001). MicroRNA primer design and detection methods were performed as previously described (Huang, C.L. et al. Disabled-2 is required for mesoderm differentiation of murine embryonic stem cells. Journal of cellular physiology 225, 92-105 (2010)).

### Immunoblot analysis

Whole cell protein was lysed using modified radioimmunoprecipitation assay (RIPA) buffer and subjected to immunoblotting using standard methodology. Membranes were screened with specific *anti*-Myocardin, *anti*-KLF4, *anti*-α-SMA or *anti*-calponin antibodies. To confirm uniform protein loading, the membranes were stripped and rescreened with either anti- -actin or anti-β-lamin antibody, as indicated.

### Single cell contraction assay

Cells cultured on glass bottom plates (MatTek Corporation) were washed twice with Krebs-Henseleit Buffer, pH 7.2-7.4 (KHB: 120 mM NaCl, 4.8 mM KCl, 2 mM CaCl₂, 1.2 mM MgSO₄, 1.2 mM KH₂PO₄, 25 mM NaHCO₃ and 5 mM Glucose). Contraction assays were performed in the presence or absence of either KCl (60 mM, Sigma) or angiotensin II (AngII; 10 nM; Bachem). Analysis was performed using ImageJ software (NIH, Bethesda, MD) to estimate the cell surface area. The change in cell surface area following addition of KCl or AngII was calculated as percentage contraction.

### Ca²⁺ signalling analysis

PPC and SMCs were grown to approximately 60% confluency on poly-D-lysine (Sigma) coated glass-bottom tissue culture dishes. On the day of analysis, the cells were washed twice with 1 mL KHB. The cells were then incubated with the intracellular ratiometric calcium indicator, fura-2 acetoxymethyl ester (3 µM in 200 µL KHB) at 22°C for 30 min, in the dark, followed by two washes with KHB. The cells were incubated with 1mL KHB at 22°C for a further 30 min to allow de-esterification of fura-2 acetoxymethyl esters by intracellular esterases. Cytoplasmic Ca²⁺ levels in these cells were assessed using epifluorescent videomicroscopy. Fura-2 loaded cells were maintained at 37°C in 1 mL KHB and challenged with 60 mM KCl after 50 sec of baseline recording. Changes in the mean fura-2 ratio (340 nm/380 nm) were recorded over 6 min using an Olympus IX51 inverted epifluorescence microscope equipped with a 75-W xenon arc lamp (Cairn), an Optiscan monochromator (Prior), and an Orca-ER charged-coupled device (CCD) camera (Hamamatsu), using an Olympus UplanF1 1.3 NA 100x oil-immersion objective. The ratiometric image of fura-2 following alternate excitation at 340 and 380 nm and measurement of emission (peak at approximately 510 nm) with a 400-nm cut off filter was collected and analysed with Andor iQ Bioimaging software, version 1.9. Mean fura-2 ratios were collected from regions of interest corresponding to the diameter of each individual cell. The exposure time of the images was 500 ms, with an incorporated interval time of 1.1 s. Each experiment was repeated a minimum of three times.

### TEVG iSMC seeding and scaffold insertion in vivo

Biodegradable poly-L-lactide (PLLA) tubular scaffolds were constructed from nonwoven fibre (300 mg/ml density, Biomedical Structures) with 1 mm internal diameter and 300 µm wall-thickness. Scaffolds were coated with 100 µl of fibronectin (20 µg/ml, Sigma) solution and dried in sterile conditions. iSMC (myocardin/GFP⁺, 2 X 10⁶) were suspended in 50 µl SmGM-2 medium with TGFβ1 (5 ng/ml) and then seeded onto the PLLA with regular rotation of the tubular scaffold. After 2 weeks culture, the ~1 cm iSMC seeded tubular scaffolds were utilized for replacement of abdominal aorta in a rodent model.

All animal experiments were approved by Animal Ethics Experimentation Committee (AEEC) of University College Cork and were performed under license from the Department of Health and Children. Prior to surgery, the scaffolds were coated externally using a layer of fibrin gel with 30 µl of fibrinogen (200 mg/ml, Calbiochem) and Thrombin (200 units/ml, Sigma). Adult male Fischer rats (weight range 300-420g) were anesthetised with an intra-peritoneal injection of Urethane (0.5 ml of 0.63 mg/ml solution), Xylazine (5mg/kg) and Ketamine (45 mg/kg) and given a loading dose of analgesia (Caprofen 5 mg/kg) by subcutaneous injection. A second dose of anaesthesia (Xylazine; 2.5mg/kg and Ketamine; 20 mg/kg), if required was administered at 45-60 mins post initial anaesthetic dose. Intravenous access was obtained by tail vein cannulation (26G, 0.64x19 mm) and animals were preloaded with 1 ml normal saline iv. The animals were maintained on a heating mat peri-operatively heated to 37°C, and temperature monitored continuously by means of a rectal probe (ATC 1000, World Precision Instruments). The surgical site was sterilised, a midline laparotomy was performed and the aorta approached by deflecting the peritoneal contents using abdominal retractors and accessed via blunt retro-peritoneal dissection. The infra renal aorta was isolated from the inferior vena cava over a 1.5 cm length between the renal arteries and the iliac bifurcation. The left genital artery or right iliolumbar artery was temporarily ligated (8-0 Nylon) allowing for sufficient vessel mobility. The mobilised aorta was cross clamped, arteriotomy performed between the clamps and a 5 mm segment of aorta excised. Heparinised saline (50 U/ml) was used to flush any thrombus out. The cellularised scaffold was interposed and anastomosed in situ using an operating microscope and 9-0 Nylon interrupted sutures. The external anastomoses and the length of the scaffold were encased in a fibrin matrix composed of 300 µl of fibrinogen (200 mg/ml) and Thrombin (200 units/ml) to allow sealing before the clamps were removed, and pressure applied for 3 minutes to ensure no bleeding from around the anastomosis sites. 1 ml of saline was injected intravenously prior to cross clamp removal, with a further 1 ml injected subcutaneously. Patency of the anastomosis was confirmed by flow testing distal to the anastomosis, by assessing capillary refill time in the hind limb paw and by recovery of normal lower limb function on recovery from anaesthesia. The peritoneum and abdominal musculature was closed with 3-0 Vicryl and the skin was closed with 4-0 monocryl. The animals were allowed to recover on heating blankets. Post operatively the animals were housed in individual cages and fed ad libitum with a 12 hour light dark cycle. Drinking water was supplemented with aspirin at 300 mg/L and the animals were monitored daily for signs of pain and any problems with hind limb mobility. Animals were sacrificed by pentobarbital overdose at 2 weeks and the scaffold harvested and processed for histological analysis.

### Tissue processing of TEVG and histological analysis

Explanted grafts were fixed in ice-cold 4% paraformaldehyde (PFA; Sigma) and embedded in paraffin and/or OCT (CellPath) for sectioning. Sections (5 µm thick) stained with Masson's trichrome were first placed in a Coplin jar containing Bouin's solution for 15 minutes at 56°C before being washed under running tap water. The slides were immersed in Hematoxylin Z solution for 15 minutes and then washed again under tap water for 15 miuntes before being rinsed in distilled water. Sections were then stained with a Ponceau 2R/Acid Fuchsin stain for 2 minutes and dipped in distilled water to remove any residual stain. Slides were treated with phosphtotungstic acid solution for 5 minutes and counter stained with Aniline blue for 3 minutes. Finally, the slides were immersed in 1% Acetic acid solution before rinsing in distilled water. Slides were dehydrated and mounted with DPX and covered with a cover slip. Tissue images were acquired using a Nikon CCD camera (DXM 1200C) attached to a Nikon microscope (ECLIPSE 80i).

### Mesenchymal differentiation of PPC

To assess whether hPPCs have mesenchymal differentiation potential, adipogenesis, osteogenesis and chondrogenesis differentiation assays were performed. Human primary mesenchymal stem cells (hMSC) were used as a positive control and were a kind gift from Dr Mary Murphy (REMEDI, NUI Galway, Ireland). The hMSC were maintained in DMEM high glucose media with 10 % fetal bovine serum and penicillin/streptomycin.

For osteogenesis, hPPC and hMSC were plated onto 6-well plates and once confluent, the maintenance media was replaced with complete Stempro osteogenesis media (Invitrogen) and cultured for 10 days with a media change at 5 days. The cells were stained using an alkaline phosphatase staining kit (Sigma Diagnostics) according to the manufacturers' instuctions.

For adipogenesis, hPPC and hMSC were cultured on 6-well plates in their appropriate maintenance media (EGM-2 for hPPC, DMEM HG + 10% FBS and P/S for hMSC). Once confluent, the media was changed to complete StemPro Adipogensis media (Invitrogen) and incubated for three days. The media was then replaced by maintenance media and incubated for one day. The cycle of induction and maintenance media replacement was repeated twice for a total of three cycles. For Oil Red O lipid staining, the cells were fixed with 10% Neutral buffered Formalin for 10 min at room temperature. Oil Red O solution was then applied and incubated for 5 mins, after which 60 % isopropanol was used to remove excess stain. The cells were then rinsed with tap water and then counterstained with haematoxylin before imaged by light microscopy.

For chondrogenesis, hPPC and hMSC were trypsinized and resuspended in 15 ml Falcon tubes with a total of 2.5 x 10⁵ cells/tube and centrifuged at 300 x g for 5 mins at 4°C. The supernatant was carefully aspirated and replaced with complete StemPro chondrogenesis media (Invitrogen) which was replaced every two days up to 14 days. The culture pellets when formed were then fixed overnight in 4% paraformaldehyde, embedded in OCT compound and cut into 5 µM thick sections with a cryostat (Leica). Slides were stained for glycosaminoglycans with toluidine blue by sequential 2 min incubations with 100%, 95%, 70% ethanol and water. The slides were then incubated with toluidine blue O (Thermo Scientific) solution (0.035% in PBS pH8.0, 60°C) for 5 mins, and then counterstained with haematoxylin. The slides were mounted with DPX (Sigma) and imaged using light microscopy.

### Chromatin immunoprecipitation (ChIP) assays

ChIP assays in PPC were performed as previously described (Turner, E.C. & Kinsella, B.T. Regulation of the human prostacyclin receptor gene by the cholesterol-responsive SREBP1. Journal of lipid research 53, 2390-2404 (2012). Chromatin protein complexes were immunopreciptiated with 3 µg *anti*-KLF4, *anti*-KLF5 or *anti*-KLF2 antibodies, whereas negative control/input DNA was immunoprecipitated with either normal rabbit IgG or no antibody. Semi-quantitative RT-PCR amplification and quantitative real-time PCR were both performed to amplify the region of the myocardin promoter containing the three KLF4 sites using the following primer sets:
forward 5'-CCTGACCATGAAAACTGTGAGATA-3' (SEQ ID 8) and reverse 5'-GCTGGTCTTGAACTCCTGCGCTCA-3' (SEQ ID 9);
forward 5'-CAGATGCACAAATAACTCTGGGTC-3' (SEQ ID 10) and reverse 5'-GACAAATGTGACACGGCGCATCTG-3'(SEQ ID 11);
forward 5'-TTCCCTTTCGCCGGCAGAAGCC-3' (SEQ ID 12) and reverse 5'-GAACCGGCTCTTAACTCTTTGCG-3'(SEQ ID 13).

Primers for the negative control region were as follows:
forward 5'-GAGTAGCCTGGCAAGGAAAACATAC-3' (SEQ ID 14) and reverse 5'-GGCTTGGAATATCTATGGTTATAAAT-3' (SEQ ID 15).

For all ChIP-based experiments, PCR (semi-quantitative and quantitative) data presented is obtained from at least 3 independent ChIP immunoprecipitations.

### Data analysis

Statistical analysis of differences were carried out using a Students' t-test and 1-way or 2-way ANOVA followed by post-hoc Dunnett's multiple comparison t tests, as indicated, throughout employing GraphPad Prism (version 4.00). P-values of less than or equal to 0.05 were considered to indicate statistical significance. As relevant, single, double and triple symbols signify p ≤ 0.05, < 0.01 and ≤ 0.001, respectively, for post-hoc Dunnett's multiple comparison t-test analyses.

## Claims

1. An induced smooth muscle-like cell (iSMC) prepared by reprogramming a primitive progenitor cell comprising introducing into the cell an agent capable of increasing the expression of myocardin in the primitive progenitor cell.

2. An induced smooth muscle-like cell of Claim 1 in which the agent is a nucleic acid construct comprising a KLF4 knockdown agent, an artificial myocardin-specific transactivator, or a myocardin transgene typically under the control of a transcriptional promotor.

3. An induced smooth muscle-like cell of Claim 2 in which the agent is a nucleic acid construct comprising a KLF4 knockdown agent, and in which the KLF4 knockdown agent is a shRNA molecule.

4. An induced smooth muscle-like cell of Claim 2 or 3 in which the nucleic acid construct is provided in the form of a viral vector.

5. A method for preparing an induced smooth muscle cell (iSMC) by reprogramming a primitive progenitor cell comprising introducing into the cell an agent capable of increasing the expression of myocardin in the primitive progenitor cell.

6. A method as claimed in Claim 1 in which the agent is a nucleic acid construct comprising a KLF4 knockdown agent, an artificial myocardin-specific transactivator, or a myocardin transgene typically under the control of a transcriptional promotor.

7. A method as claimed in Claim 6 in which the agent is a nucleic acid construct comprising a KLF4 knockdown agent, and in which the KLF4 knockdown agent is a shRNA molecule.

8. A method as claimed in Claim 6 or 7 in which the nucleic acid construct is provided in the form of a modified RNA construct or a viral vector encoding myocardin.

9. An induced smooth muscle-type cell (iSMC) according to any of Claims 1 to 4, for use in a method of cell based *in-vivo* tissue regeneration in a mammal.

10. An induced smooth muscle-type cell (iSMC) according to any of Claims 1 to 4, for use in a method of autologous or allogeneic cell based *in-vivo* tissue regeneration in a mammal.

11. An induced smooth muscle-type cell (iSMC) according to any of Claims 1 to 4, for use in autologous or allogeneic cell based *in-vivo* regeneration of the vasculature tissue, respiratory tract tissue, gastrointestinal tract tissue, and genito-urinary tract tissue where the regenerative scaffold is constituted by biodegradable or non-biodegradable polymer.

12. An induced smooth muscle-type cell (iSMC) according to any of Claims 1 to 4, for use in a method of treatment of chronic vascular occlusion in a mammal, in which an intravascular cell delivery device comprising the induced smooth muscle-type cell is implanted into the vasculature at or close to the vascular occlusion.

13. 1 An induced smooth muscle-type cell (iSMC) according to any of Claims 1 to 4, for use in a method of treatment of chronic vascular occlusion in a mammal, in which an intravascular cell delivery device is comprised of stainless steel, nitinol, cobalt chromium or tantalum or of a biodegradable or non-biodegradable polymer.

14. A bioactive tissue repair construct comprising a tissue repair construct seeded with induced smooth muscle cells (iSMC's) of any of Claims 1 to 4.

15. A bioactive tissue repair construct as claimed in Claim 13 in which the tissue repair construct is an intravascular cell delivery device.

16. Mammalian tissue generated *in-vitro* by seeding a tissue repair construct with induced smooth muscle cells according to any of Claims 1 to 4 and incubating the seeded tissue repair construct in a suitable growth medium for a period of time sufficient to allow the cells grow and generate the mammalian tissue.


SEQUENCE LISTING

<110>  UNIVERSITY COLLEGE CORK, NATIONAL UNIVERSITY OF IRELAND,
       CORK

<120>  Induced smooth muscle-type cells, methods for the production
       thereof, and uses thereof in cell-based tissue regeneration and
       treatment of vascular occlusion

<130>  P11534EP01

<150>  EP15154636.3
<151>  2015-02-11

<160>  15

<170>  PatentIn version 3.5

<210>  1
<211>  28
<212>  DNA
<213>  Artificial Sequence

<220>
<223>  Isl1 PCR forward primer

<400>  1
gatggtaccc tcaactaaat gaggctac                                          28


<210>  2
<211>  29
<212>  DNA
<213>  Artificial Sequence

<220>
<223>  Isl1 PCR reverse primer

<400>  2
attgtcgact tgtaagaggg agtaatgtc                                         29


<210>  3
<211>  25
<212>  DNA
<213>  Artificial Sequence

<220>
<223>  Myocardin PCR forward primer

<400>  3
aaagacctga attctatcaa taacc                                             25


<210>  4
<211>  30
<212>  DNA
<213>  Artificial Sequence

<220>
<223>  Myocardin PCR reverse primer

<400>  4

acaacccagg atccatccaa ctctccgtga                                    30

<210>  5
<211>  32
<212>  DNA
<213>  Artificial Sequence

<220>
<223>  Myocardin cDNA PCR forward primer

<400>  5
aagctagcta gcatgacact cctggggtct ga                                 32

<210>  6
<211>  36
<212>  DNA
<213>  Artificial Sequence

<220>
<223>  Myocardin cDNA PCR reverse primer

<400>  6
tattcttacg ccggcgtcta gagtctgctg tacatt                             36

<210>  7
<211>  19
<212>  DNA
<213>  Artificial Sequence

<220>
<223>  KLF4 shRNA target sequence

<400>  7
ggacggctgt ggatggaaa                                                19

<210>  8
<211>  24
<212>  DNA
<213>  Artificial Sequence

<220>
<223>  KLF4 site PCR Forward Primer

<400>  8
cctgaccatg aaaactgtga gata                                          24

<210>  9
<211>  24
<212>  DNA
<213>  Artificial Sequence

<220>
<223>  KLF4 site PCR reverse primer

<400>  9
gctggtcttg aactcctgcg ctca                                          24

<210>  10
<211>  24
<212>  DNA
<213>  Artificial Sequence

<220>
<223>  KLF4 site PCR forward primer

<400>  10
cagatgcaca aataactctg ggtc                                              24


<210>  11
<211>  24
<212>  DNA
<213>  Artificial Sequence

<220>
<223>  KLF4 site reverse primer

<400>  11
gacaaatgtg acacggcgca tctg                                              24


<210>  12
<211>  22
<212>  DNA
<213>  Artificial Sequence

<220>
<223>  KLF4 site forward primer

<400>  12
ttccctttcg ccggcagaag cc                                                22


<210>  13
<211>  23
<212>  DNA
<213>  Artificial Sequence

<220>
<223>  KLF4 site PCR reverse primer

<400>  13
gaaccggctc ttaactcttt gcg                                               23


<210>  14
<211>  25
<212>  DNA
<213>  Artificial Sequence

<220>
<223>  KLF4 negative control region forward primer

<400>  14
gagtagcctg gcaaggaaaa catac                                             25


<210>  15
<211>  26
<212>  DNA

<213>  Artificial Sequence

<220>
<223>  KLF4 negative control region reverse primer

<400>  15
ggcttggaat atctatggtt ataaat                                          26